# EUROPEAN PATENT SPECIFICATION

(11) **EP 0 671 924 B1**
(45) Date of publication and mention of the grant of the patent: **10.12.2003**
(21) Application number: 93922681.7
(22) Date of filing: 08.09.1993
(51) Int. Cl.: A61K 35/44, A61K 31/35, A61K 31/704, C07H 15/24, C07J 17/00, A61P 9/00

(54) **DIGITALIS-LIKE COMPOUNDS**
DIGITALISARTIGE VERBINDUNGEN
COMPOSES SEMBLABLES A LA DIGITALINE

(30) Priority: 10.09.1992 IL 10313292
(43) Date of publication of application: 20.09.1995
(73) Proprietor: YISSUM RESEARCH DEVELOPMENT COMPANY OF THE HEBREW UNIVERSITY OF JERUSALEM, Jerusalem 91042 (IL); KOHN, Kenneth I., Troy, MI 48099 (US)
(72) Inventor: LICHTSTEIN, David, 97 735 Jerusalem (IL); DEUTSCH, Joseph, 96 348 Jerusalem (IL); GATI, Irit, 93 843 Jerusalem (IL)
(74) Representative: Wharton, Peter Robert
(86) International application number: US9308393
(87) International publication number: WO94005305

(56) References cited:
- GB-A- 1 342 761
- US-A- 4 380 624
- US-A- 4 780 314
- Biochimica et Biophysica Acta, Volume 693, issued 1982, SEN et al., "Characterization of Partially Purified (Na+ and K+)-ATPase from Porcine Lens", pages 34-44, see entire document.
- Japanese Journal of Ophthalmology, Volume 33, issued 1989, "Characterization of Peptide Inducing Cataractogenesis in Lens of Hereditary Cataractous Rat (ICR/f Rat)", pages 348-357, see entire document.
- Archives of Biochemistry and Biophysics, Volume 151, No. 1, issued July 1972, W.B. VAN WINKLE et al., "The Nature of the Transport ATPase-Digitalis Complex: III. Rapid Binding Studies and Effects of Ligands on the Formation and Stability of Magnesium Plus Phosphate-Induced Glycoside-Enzyme Complex", pages 85-92, see especially page 85, first paragraph.
- Vision Research, Volume 21, issued 1981, P. RUSSELL et al., "Properties of a Na-K ATPase Inhibitor in Cultured Lens Epithelial Cells", pages 37-39, see entire document.
- European Journal of Biochemistry, Volume 216, issued 1993, LICHTSTEIN et al., "Identification of Digitalis-Like Compounds in Human Cataractous Lenses", pages 261-268, see entire document.

## Description

The Na⁺,K⁺-ATPase (E.C.3.6.1.3) is an integral plasma membrane protein responsible for the maintenance of Na⁺ and K⁺ concentration gradients in all eukaryotic cells. Since Na⁺,K⁺-ATPase has a high-affinity receptor for digitalis steroids, it has been postulated that there are endogenous ligands for these receptors which regulate the Na⁺,K⁺-pump activity. Indeed, based on their ability to inhibit [³H]-ouabain binding and Na⁺,K⁺-ATPase activity, digitalis-like compounds (DLC) have been shown to be present in the brain [Haupert, G.T., Jr. & Sancho, J.M. (1979) Proc. Natl. Acad. Sci. USA 76:4658-4660;Lichtstein, D. & Samuelov, S. (1980) Biochem. Biophys. Res. Com. 96, 1518-1523; Akagawa, K., et al., (1984) J. Neurochem. 42:775-780], heart [De Pover, A., et al. (1982) Biochem. Pharmacol. 31:267-271], adrenal [Tamura, M., et al., (1988) Biochemistry 27:4244-4253], plasma [Kramer, H.J., et al. (1977) Kidney International 12:214-219; Gruber, K.A. et al. (1980) Nature 237:743-745; Moreth, K., et al. (1986) Klinische Wochenschrift 64:239-244; Dasgupta, A., et al. (1987) Biochem. Biophys. Res. Commun. 148:623-628], CSF [Halperin, J., et al. Proc. Natl. Acad. Sci. USA 80:6101-6104; Lichtstein, D., et al. (1985) Brain Res. 325:13-19] and urine [Cloix, J.F. et al. (1987) Can. J. Physiol. Pharmacol. 65:1522-1527; Goto, A., et al. (1990) Biochem. Biophys. Res. Commun. 173:1093-1101] of mammals and in the skin [Flier, J., et al. (1980) Science 208:503-505] and plasma [Lichtstein, D., et al. (1986) Life Sciences 38:1261-1270] of toads. Several substances have been proposed as the DLC, including unsaturated fatty acids [Bidard, J.N., et al. (1984) Biochim. Biphys. Acta 769:245-252; Tamura, M., et al. (1985) J. Biol. Chem. 260:9672-9677; Kelly, R.A., et al. (1986) J. Biol. Chem. 261:11704-11711], hydroxy unsaturated fatty acids [Lichtstein, D., et al. (1991) J. Endocrinol. 128:71-78,], lysophosphatidylcholines [Tamura, M., et al. (1987) Biochemistry 26:2797-2806], dopamine [Clarkson, E.M. & De Wardner, H.E. (1985) Clinical and Experimental Hypertension Part A, A7, 673-683], dehydroepiandrosterone sulfate [Vasdev, S., et al. (1985) Res. Commun. Chem. Path. and Pharmacol. 49:387-399], lignan [Fagoo, M., et al. (1986) Biochem. Biophys. Res. Commun. 134:1064-1070] and ascorbic acid [Ng, Y.C., et al. (1985) Biochem. Pharmacol 34:2525-2530]. However, none of these compounds appears to be the natural ligand of the digitalis receptor of the Na⁺,K⁺-ATPase because of their limited specificity and affinity. Bufodienolides, which resemble the structure of the plant cardiac glycosides, have been identified in the plasma, brain and other tissues of toads [Flier, J., et al. (1980) ibid.; Lichtstein, D., et al. (1986) ibid.; Meyer, K. & Kinde, H. (1971) Collection of Toad Venoms and Chemistry of the Toad Venom Steroids. Vol. 2 p. 521-552, in Venomous Animals and their Venoms, Eds W. Bucherl & E. Buckley. New York Academic Press; Lichtstein D., et al. (1991) Biochem. Biophys. Acta. 1073:65-68]. However, the presence of bufodienolides in mammals has not yet been demonstrated. Recently, ouabain [Hamlin, J.M., et al. (1991) Proc. Natl. Acad. Sci. USA 88:6257-6263] and digoxin [Goto, A., et al. ibid.] have been identified in mammalian tissues but their synthesis by mammals has not been demonstrated. Numerous studies have implicated DLC in the regulation of sodium and water homeostasis and pregnancy as well as pathological states such as hypertension and renal and hepatic failure [for review see De Wardener, H.E. & Clarkson, E.M. (1985) Physiological Reviews 65:658-759; Haber, E. & Haupert, G.T. (1987) Hypertension 9:315-324; Wechter, W.J. & Benaksas, Prog. Drug Res. 1990, 34:231-260].

Availability of endogenous digitalis-like compounds and the possibility of their administration as therapeutic agents may result in advantageous treatment of various pathological conditions in which their involvement is implied. such compounds may be used as cardiotonic agents, increasing the intensity of heart muscle contractions, as vasoactive agents, elevating blood pressure and as natriuretic/diuretic agents, increasing the excretion of sodium into the urine and thus increasing urine volume. In view of the marked involvement of Na⁺,K⁺-ATPase in the central nervous system, digitalis-like compounds may also be used as neuromodulating agents.

In search for endogenous digitalis-like compounds, the present inventors have surprisingly found that such compounds are present in human cataractous lens nuclei.

The eye lens is an avascular tissue composed of two different cell types: fibre cells that are essentially free of intracellular organelles that make up the bulk of the lens, and a single layer of epithelial cells on the anterior surface from which the fibre cells are derived [Duncan, G. & Jacob, T.J. The Eye (Davson, H., Ed.) pp.159-206, Academic Press, Orlando, FL, USA (1984). The anatomy and biochemistry of the lens as well as its development were extensively studied (for review see Duncan, G. & Jacob, T.J. (1984) ibid.; Collier E., The Lens in: Adler's Physiology of the Eye (Moses R.A. ed) 277-303, The C.V. Mosby Company, St. Louis (1981)]. Studies of lens plasma membrane ion pumps have indicated that the lens Na⁺,K⁺-ATPase could maintain intracellular Na⁺ and R⁺ concentrations similar to those in other tissues [Sen P.C. & Pffifer D.R. (1982) Biochim. Biophys. Acta 693:33-34].

Several studies raised the possibility that a Na⁺,K⁺-ATPase inhibitor is involved in cataract formation. In 1960, a strain of mice that develops lens opacity shortly after birth was described [Nakano K., et al. (1960) Jap. J. ophthalmol. 14:196]. Further investigation of the mechanism of the development of this hereditary cataract suggested that an apparent deficiency of Na⁺,K⁺-ATPase activity in the lens may be involved [Itawa, S & Kinoshita, J H (1971) Invest. Ophthalmol. 10:504-512]. The continuation of this research led to the isolation of a "cataracteogenic factor" from mice lenses, that inhibits Na⁺,K⁺-ATPase activity [Fukui H N, et al (1978) Exp. Eye. Res. 26:1-9; Russell P, et al (1981) Vision Res. 21:37-39]. It was further demonstrated that the levels of this endogenous inhibitor are elevated in cataractous lens but it is also present in the normal lens [Russel, P, et al (1981) ibid.]. The study of Fukui et al [(1978) ibid.] as well as the recent study by Kamei and Sakai (Kamei, A & Sakai, H (1989) Jap. J. Ophthalmol. 33:348-357] strengthen the possibility that this Na⁺,K⁺-ATPase inhibitor is a peptide. However its amino acid composition and sequence have not been determined. The study by Kamei and Sakai (Kamei, A & Sakai, H (1989) Jap. J. Ophthalmol. 33:348-357] discloses that a material with inhibitory action to Na⁺, K⁺-ATPase was found in the lens of recessive hereditary cataractous rats (ICR/f rat)

According to the present invention, there is provided a human cataractous lens nuclei methanolic extract consisting essentially of substances having a molecular weight lower than 3000 daltons, which substances are characterised by the following properties:
(a) they are capable of inhibiting ATP hydrolysis by Na⁺, K⁺-ATPase;
(b) they are capable of inhibiting ouabain binding to the ouabain binding site of Na⁺, K⁺-ATPase;
(c) they are capable of cross-reacting with an antibody directed against digoxin; and
(d) they have an absorption in the UV range.
The Na⁺, K⁺-ATPase is preferably rat brain microsomal ATPase.

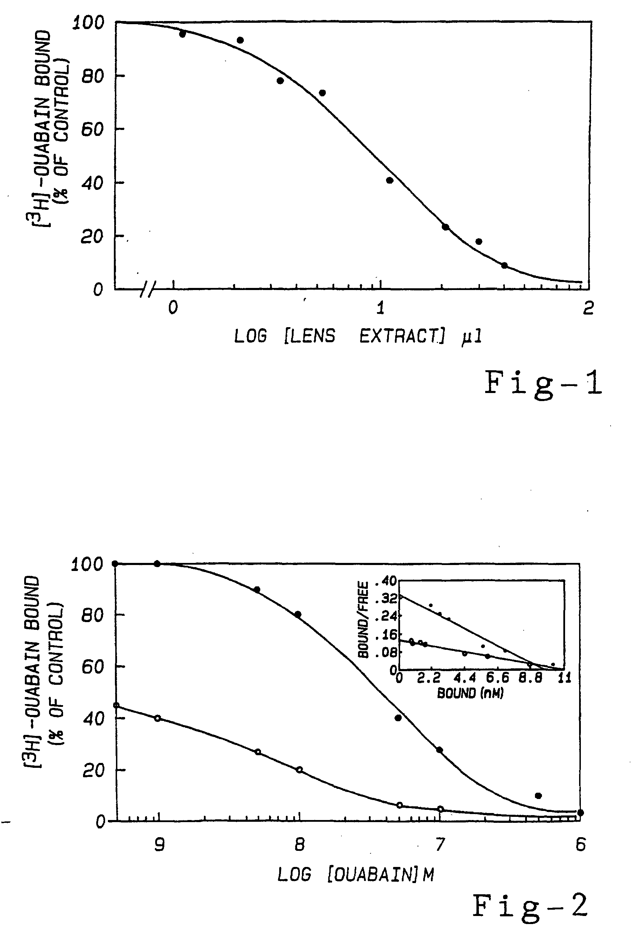

Preparation of the human cataractous lens nuclei extract according to the invention is described in detail hereinafter in the Examples. The Examples also demonstrate that the human cataractous lens extract of the invention exhibits digitalis-like activity in that is inhibits, in a dose dependent fashion, [³H]-ouabain binding to rat brain synaptosomes, it inhibits ATP hydrolysis by rat brain microsomal Na⁺, K⁺-ATPase and binds with an antibody directed against digoxin. The dose dependency of the inhibition of [³H]-ouabain and Na⁺, K⁺-ATPase activity by the human cataractous lens extract of the invention is similar to the effects of known cardiac glycosides and spans two log units for complete pharmacological effect. This is shown in Figure 1 and TABLE 1 hereafter.

The present invention further provides a compound of the general formula (I): 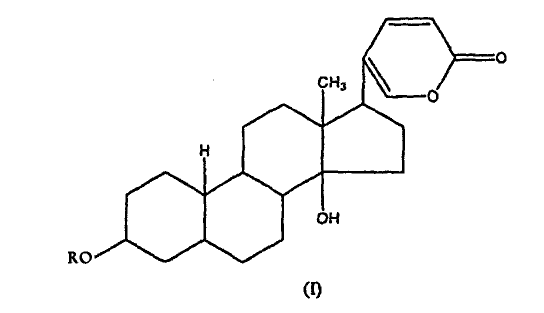 in which R is hydrogen, an amino acid, a di- or tri-peptide, or a mono-, di- or tri-saccharide.

Preferred compounds of formula I are those in which R is a hydrogen atom.

Preferred amino acids as the R substituent are threonine, glycine and alanine. Preferred di- or tripeptides as the R substituent are peptides consisting of said three amino acid residues.

Preferred saccharide as the R substituent is mannose.

The most preferred compound of formula I is 19-norbufalin and its conjugate with the tri-peptide Thr-Gly-Ala-NH₂.

The isolation and identification of compounds of general formula I are described hereinafter in the Examples, which also demonstrate their digitalis-like activity.

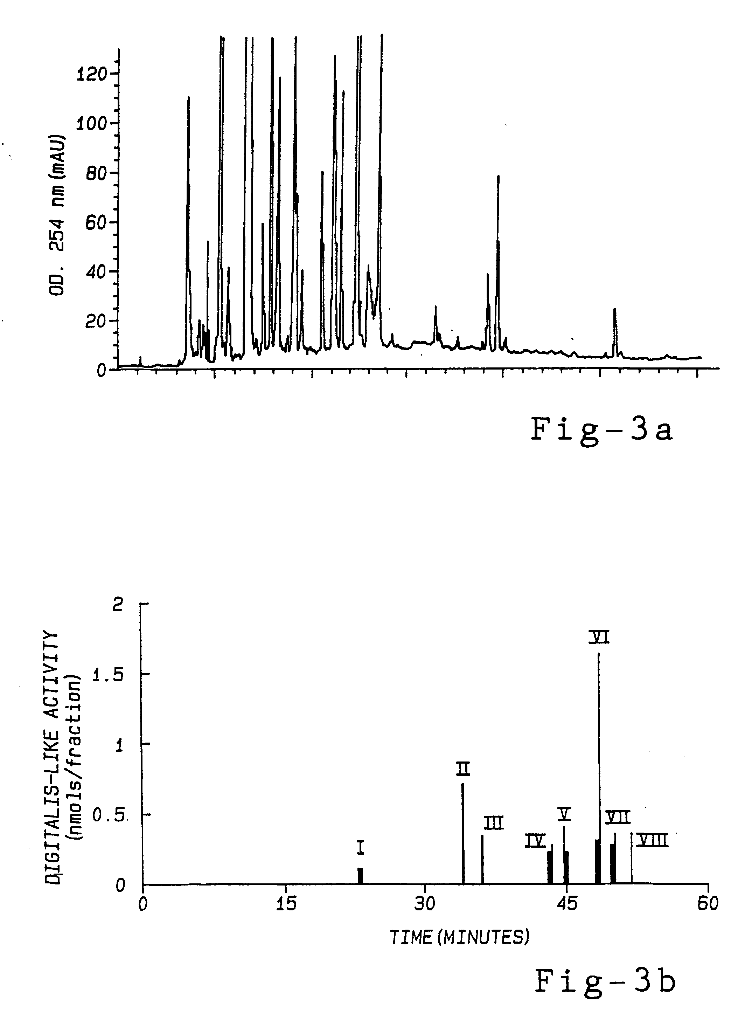

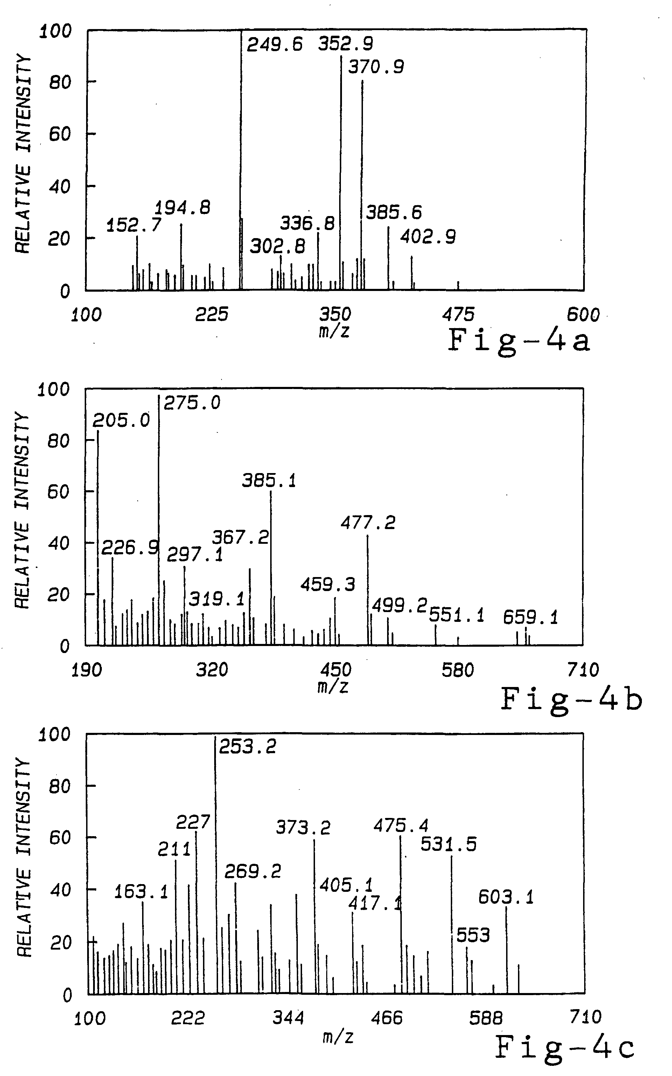

The structure of compounds of general formula I resembles the structure of some bufodienolides which were identified as normal constituents of toad skin and plasma [Flier, J, et al (1980) ibid.; Lichtstein, D, et al (1986) ibid.; Meyer, K & Kinde, H (1971) ibid.]. This is based on the following observation:
1) the mass spectrum of Fractions VI and II of the human cataractous lens nuclei extract of the present invention corresponds to the suggested dienolide structures (Fig.3);
2) the maximum UV absorbance of material in Fraction VI was 298 nm, a UV maximum characteristic of the dienolide system (TABLE 3) [Meyer, K & Kinde, H (1971) ibid.]; and
3) the digitalis-like activity characteristic of steroids as delineated above. The present invention implies that the synthesis of 6-membered cyclic unsaturated lactone steroids is not restricted to amphibian, but is also achieved by mammalian tissues.

The present invention further provides 3-hydroxy-14-en-bufa-20, 22-dienolide and its conjugate with the tri-peptide Thr-Gly-Ala-NH₂.

The invention also provides a pharmaceutical preparation comprising as an active ingredient a therapeutically effective amount of the human cataractous lens nuclei extract of the present invention and optionally a pharmaceutically acceptable carrier or diluent.

The invention also provides a pharmaceutical preparation comprising as an active ingredient a pharmaceutically effective amount of at least one compound of the present invention and optionally a pharmaceutically acceptable additive in a pharmaceutically acceptable carrier or diluent.

Preferred pharmaceutical preparations of the present invention comprise as active ingredient 19-norbufalin or its conjugate with Thr-Gly-Ala-NH₂.

Pharmaceutical preparations comprising as active ingredient suitable mixtures of at least two compounds according to the present invention are also envisaged.

The pharmaceutical preparations of the present invention may be used for the treatment of malfunctions such as cardiac arrhythmia and cardiac failure, for the induction of natriuresis and diuresis, for the constriction of smooth muscles in arterioles, causing increase in blood pressure and as neuromodulators affecting the central nervous system.

The effectivity of the pharmaceutical preparations of the present invention is based on the established biological effects of cardiac glycosides and bufodienolides and the resemblance thereto of the compounds of the present invention, which constitute the active ingredient of the pharmaceutical preparations according to the invention. This effectively is also based on the ability of the compounds of the present invention to inhibit Na⁺, K⁺-ATPase activity, as will be shown in the Examples hereafter.

The pharmaceutical preparation according to the present invention may be administered orally or parenterally in dosage unit preparations containing conventional nontoxic pharmaceutically acceptable carriers, diluents, adjuvants and vehicles as desired. The term parenteral as used herein includes subcutaneous, intravenous, intramuscular, intraarticular and intrathecal injection and infusion techniques.

Dosage unit preparations may contain daily required amounts of the compounds of the present invention or submultiples thereof to make up the desired dose. The specific therapeutically effective dose level for any particular patient will depend upon a variety of factors including the activity of the specific compound employed, the age, body weight, general health, sex, diet, time of administration, route of administration, rate of excretion, etc.

As used herein, the term "pharmaceutically acceptable carrier" means an inert, non-toxic solid or liquid filler, diluent or encapsulating material, not reacting with the active ingredients of the invention. These carriers are known to the man versed in the art.

Wetting agents, emulsifiers and lubricants, as well as colouring agents, release agents, coating agents, sweetening and flavouring agents and preservatives can also be present in the pharmaceutical preparation of the present invention. The amount of active ingredient that may be combined with the carrier materials to produce a single dosage form, will vary depending upon the patient treated and the particular mode of administration.

The present invention further provides use of human cataractous lens nuclei methanolic extract according to the present invention, or of a compound according to the present invention, in the preparation of a medicament for the treatment or prevention of cardiac malfunction or renal malfunction, or for the induction of natriuresis and diuresis, or for modulating activity of the central nervous system.

The invention may be more clearly understood with reference to the accompanying Figures, given by way of example only, wherein:
Figure 1 is a graph showing a displacement curve of [³H] -ouabain binding to rat brain synaptosomes by digitalis-like compounds (DLC) partially purified from human cataractous lenses. Control [³H] -ouabain binding in the absence of DLC was 15 pmol/mg protein.
Figure 2 is a graph similar to the graph of Figure 1 with a different horizontal scale or abscissa.
Figure 3 shows an elution profile of DLC on high performance liquid chromatography, C-18 column. Aliquots of the partially purified material obtained from the <3000 dalton cutoff filter (see Materials and Methods) were dissolved in water and injected onto a Lichrospher RP-18 column (0.4 X 25 cm, 5µm particle size) equilibrated with 80% acetonitrile and eluted with a linear 5-80% acetonitrile gradient in 60 min at a flow rate of 3 ml/min. The line represents the absorbance at 254 nm (Figure 3a). The digitalis-like activity in each fraction was determined by its ability to inhibit [³H]-ouabain binding (solid bars in Figure 3b).
Figure 4 shows chemical ionisation mass spectra of bufalin and purified material from human cataractous lens nuclei. Chemical ionisation mass spectra were determined on Finnigan TSQ 70 mass spectrometer using isobutan as the ionisation reagent as described in Materials and Methods:
   Figure 4a shows a negative chemical ionisation mass spectrum of fraction VI;
   Figure 4b shows a negative chemical ionisation mass spectra of commercial bufalin (Sigma Chemicals, St. Louis, Mo . USA);
   Figure 4c shows a positive chemical ionisation mass spectra of fraction II.

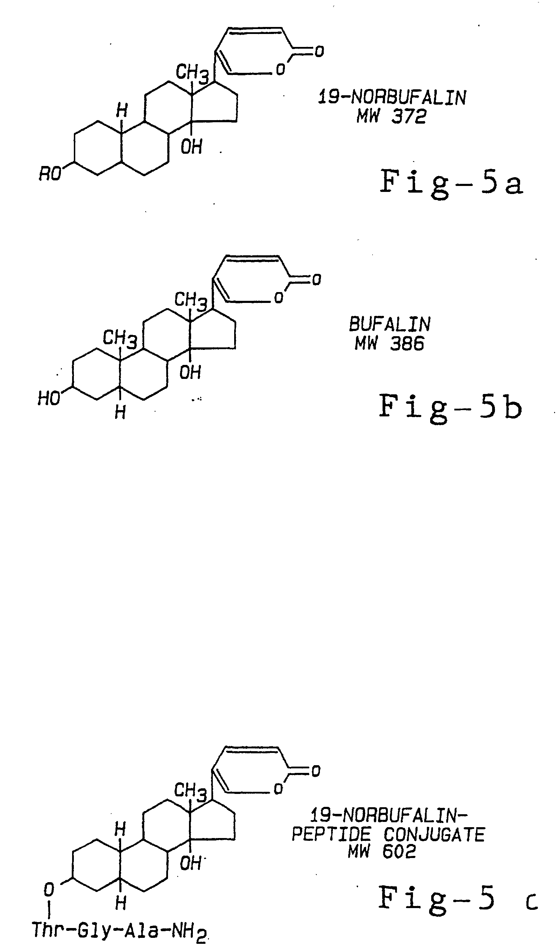

Figures 5a to 5c show postulated structures of mammalian digitalis-like compounds.

### EXAMPLES

### Materials and Methods

Lenses extraction: Human cataractous lens nuclei were obtained from patients which were medication-free, undergoing cataract surgery at the Shaarey Zedek and Hadassah Hospitals in Jerusalem. The lens nuclei were immediately frozen and kept at -20°C until used. Pooled 20-30 lens nuclei were homogenised in methanol. The homogenate was kept for 1 h at 4°C and the precipitate removed by centrifugation (28,500xg, 4°C). The methanol was evaporated and the residue dissolved in distilled water. The sample was centrifuged again under the same conditions and the aqueous supernatant separated and applied to Sep-pak C-18 columns (Waters, MA, U.S.A.). The Sep-pak columns were washed with 10 ml water and the active material was eluted by 80% acetonitrile. The acetonitrile was evaporated and the residue was dissolved in water. The sample was applied to Centricon-3 (3,000 MW cutoff) microconcentrator tubes (Amicon MA, U.S.A.) which were centrifuged for 3 h at 5,000xg. The material that passed the filter (<3000 Dalton) was collected, concentrated and applied to HPLC for fractionation (see Results) . The material obtained at this stage of purification is termed, in the present study, 'partially purified material'. Rat, rabbit and bovine lenses were extracted and partially purified in a manner identical with the human cataractous lenses extraction.

Assays for digitalis-like activity: The digitalis-like activity of each fraction obtained in the course of the purification was determined using two independent bioassays. The first assay was an assay to determine the inhibition of [³H]-ouabain binding. The binding of [³H]-ouabain to rat brain synaptosomal fractions was carried out by a conventional filtration technique as previously described [Lichtstein, D., et al. (1991) ibib.]. In brief: the reaction mixture (500 µl final volume) contained the following constituents (final concentrations): Tris-Hcl (50 mM) buffer (pH 7.4), EDTA (0.4 mM), NaCl (80 mM), MgSO4 (4 mM), ATP (2 mM, Tris, vanadium-free), [³H]-ouabain (32 nM, 19.5 Ci/mmol, New England Nuclear, Dreisich, Germany) and other additions as indicated. Reactions were initiated by the addition of 200 µl crude synaptosomal fraction resulting in a final protein concentration of 200 µg/reaction. Following incubation for 1 h at 37°C, the reactions were terminated by the addition of 3 ml cold (4°C) Tris-HCl (50 mM, pH 7.4) and filtered over Whatman GF/B filters (Tamar Inc., Jerusalem, Israel). Filters were washed twice with 3 ml of the same Tris buffer, dried and assayed for radioactivity in a Packard liquid scintillation counter at a counting efficiency of approximately 50%. Specific binding was calculated by subtracting the binding observed in the presence of 100 µM unlabelled ouabain from that observed in the absence of unlabelled ouabain. Specific binding represented 93% of the total binding under control conditions. All samples tested for their ability to inhibit [³H]-ouabain binding were analysed previously for Na⁺ and K⁺ levels. In all cases K⁺ final concentrations were too low (<10 µM) to affect the binding characteristics.

The second assay was an assay to determine the inhibition of Na⁺,K⁺-ATPase activity, Na⁺,K⁺-ATPase activity in rat brain microsomal fraction was determined as previously described (Lichtstein, D., et al. (1991) ibid.]. Enzyme activity was measured by the colourimetric determination of inorganic phosphate after the incubation of microsomes (30 µg protein/reaction) at 37°C in a solution (final volume 500 µl) containing (final concentrations) Tris-HCl (50 mM, pH 7.4), NaCl (100 mM), KCl (10mM), MgCl₂ (4 mM) and ATP (2 mM, Tris, vanadium free). After 10 min preincubation, the ATP was added to initiate the reaction. Reactions were terminated by the addition of 100 µl 5% trichloroacetic acid and the precipitate was removed by centrifugation. Ouabain-insensitive ATPase activity was determined by the addition of ouabain (1mM) to the assay mixture.

Measurement of digoxin-like immunoreactivity: Radioimmunoassay was performed with digoxin RIA kit obtained from Amersham International (Amersham, Buckinghamshire, England). Assay conditions were as recommended by the manufacturer.

High-performance liquid chromatography (HPLC): Reversed-phase HPLC were run on a Lichrospher RP-18 column (0.4X25 cm, 5 µm particle size, Merck, Darmstadt, Germany) using the Hewlett Packard HPLC pump (Model 1050) connected to computerised diode array detector (Model 1040M). The columns were pre-equilibrated with water containing 0.1% trifluoroacetic acid before sample application and developed with a linear gradient of acetonitrile of 0-80% acetonitrile containing 0.1% trifluoroacetic acid for 60 min at a flow rate of 1 ml/min. The elution of the compounds from the columns was monitored at various wavelengths using the diode array detector and each sample was measured for digitalis-like activity as described above.

Mass spectrum measurements: Mass spectra were recorded by negative and positive chemical ionisation on a Finnigan TSQ 70 mass spectrometer using isobutane as the ionisation reagent. The samples were inserted by direct inlet with a chemical desorption sampler. The samples were heated from 25 to 250°C and the spectra were automatically recorded.

Na⁺,K⁺ and protein determinations: Na⁺,K⁺ were determined by atomic absorption using a Perkin-Elmer atomic absorption spectrometer (Norwalk, Connecticut, U.S.A.). Protein concentration was determined by the colourimetric method [Lowry, O.H., et al. (1951) J. Biol. Chem. 36:265-275].

### Results

### Digitalis-like activity in human cataractous lenses and normal animal lenses:

The inhibition of [³H]-ouabain binding to rat brain synaptosomes by partially purified lens extract is demonstrated in Figure 1. The dose response of this inhibition spans two log units as expected from mass-action law for ligand receptor interaction. Assuming that the compound has the same affinity to the ouabain receptor as ouabain, the levels of DLC in the lens could be estimated. In 71 different pooled lens nuclei extracts, 15 ± 7 nmol/gr wet weight (Mean ± S.E.M. ouabain equivalents) were found (the large variability in DLC levels in the cataractous lens is referred to in the following discussion).

Furthermore, the partially purified material from human cataractous lens extract inhibited, dose dependently and specifically, the ATP hydrolysis by rat brain microsomal Na⁺,K⁺-ATPase (Table 1). Importantly, extract concentration that caused 80% inhibition of Na⁺,K⁺-ATPase activity had only marginal effect on Mg⁺⁺-ATPase activity.

Human cataractous lens nuclei were extracted and partially purified as described in Materials and Methods. ATPases activity were performed on rat brain microsomal membrane fraction. Values are from a single experiment conducted in triplicates.

The partially purified material from human cataractous lenses was also tested for its ability to cross react with digoxin antibody in comparison to ouabain and bufalin (Table 2). The three compounds cross reacted with the antibody resulting in a cross reactivity of 0.012, 0.045 and 0.022% for ouabain, bufalin and the lens extract, respectively. It should be pointed out that the cross reactivity calculation for the compound in the lens were done under the following assumptions: (a) that the compound has an identical affinity to ouabain in the [³H]-ouabain binding assay; (b) that it has a MW of 372 (see Table 2); and (c) that a single compound is involved.

Digoxin radioimmunoassay was performed using commercially available kit. The concentration of the partially purified lens DLC was determined from its ability to inhibit [³H]-ouabain binding assuming identical affinity to the ouabain receptor as ouabain and MW of 372.

In order to eliminate the possibility of contamination of the extract with cardiac glycosides or bufodienolides present in the laboratory, the extraction and purification was repeated three times in a different laboratory (different building) with identical results. In additional three experiments, water was substituted for lens extract and was taken through the extraction and purification procedures. No digitalis-like activity (inhibition of [³H]-ouabain binding and Na⁺,K⁺-ATPase activity) was apparent.

Taken together, the effects of the partially purified cataractous lens extract on [³H]-ouabain binding, Na⁺,K⁺-ATPase and digoxin antibody, strongly suggest the existence of DLC in this tissue.

### Purification of digitalis-like compounds:

In the present study approximately 600 human cataractous lens nuclei were used. The lenses were homogenised in methanol and extracted and partially purified as described in Materials and Methods. The material obtained was redissolved in distilled water and applied to fractionation on HPLC.

The elution profile of the samples from reverse phase C-18 column is shown in Figure 3. The digitalis-like activity was fractionated into eight peaks of activity designated I to VIII (Figure 3b). Each of these peaks inhibits, in a dose dependent manner, [³H]-ouabain binding to rat brain synaptosomes and rat brain microsomal Na⁺,K⁺-ATPase activity (data not shown). Some chemical characteristics of these peaks are summarised in Table 3. Most of the digitalis-like activity was obtained in peak VI and peak II. While peak VI shows a clear maximum of UV absorbance at 298 nm, peak II shows a maximum at 289 nm (Table 3).

The maximum absorbance of each peak of activity in the chromatogram (Figure 3) was determined using the diode array detector. % digitalis-like activity was determined by measuring the ability of each fraction to inhibit [³H]-ouabain binding to rat brain synaptosomes. The reactivity of each fraction to diazomethane was measured by treating the partially purified extract with etheral solution of diazomethane prior to the fractionation on HPLC.

In order to shed light on the chemical nature of the compounds involved, an experiment was conducted in which the lens extract was treated with diazomethane prior to its separation on HPLC. The rational was that if free carboxylic acid is involved in the structure the compound will either lose its biological activity or be shifted in the chromatogram. As can be seen in Table 3, a variety of results were obtained for the different peak. Peak VII disappeared from the chromatogram, peak IV was significantly shifted and some peaks (I, II, VI, VIII) were not affected by the treatment. These results may indicate that a mixture of compounds with similar biological activity is present in the cataractous lens.

### Structure Identification:

Identification of the structure of the endogenous compounds was performed on fractions VI and II. As mentioned above, fraction VI, with the retention time of 46.5-47.5 min., shows a UV absorbance with lambda max of 298 nm suggesting that a bufodienolide structure may be present in this fraction. The mass spectral analysis using negative chemical ionisation of this fraction is shown in Figure 4a (M-1 ions are shown). It reveals a small peak at m/z 385 and two larger peaks at m/z 371 and 353. The peak at 385 may correspond to cholesterol (MW 386) which is known to be present in the lens but may also correspond to the bufodienolide bufalin. In addition to cholesterol, two other bufodienolides, 19-norbufalin (MW 372) and its dehydrated derivative (MW 354), may be present in this fraction (Figure 4a). The large peak at m/z 249 (Figure 4a) is due to a sodium-glycerol cluster ion G₂Na₃. For comparison, the mass spectrum of bufalin, obtained also in negative chemical ionisation, is shown in Figure 4b. It shows a peak at m/z 385 corresponding to the (M-H)⁻ ion and a peak at m/z 477 due to the bufalin-glycerine adduct, without undergoing further fragmentation. Glycerin trimer (m/z 275), tetramer (m/z 367) and sodium-glycerine cluster (m/z 205) are also present in this mass spectrum.

The mass spectrum of the more polar fraction (fraction II, retention time 33.5-34.5 min.) further supports conclusions. The mass spectrum, measured by positive chemical ionisation mode which shows mainly protonated ions (M+1 ions), is depicted in Figure 4c. It shows peaks at m/z 373, 475, 531 and 603 au. Analysis of this mass spectrum suggests that the 19-norbufalin (MW 372) is bound in this fraction to a small peptide (Thr-Gly-Ala, 373+101+57+72=603) or fragments of this peptide (373+101+58=532 and 373+102=475) (Figure 4c). The 253 peak, due to the sodium-glycerol cluster (G₂Na₃) is the most abundant peak in this spectrum. Due to the minute quantities of material available, it was not possible to conclude whether fraction II is a mixture of these derivatives or a homogenous compound.

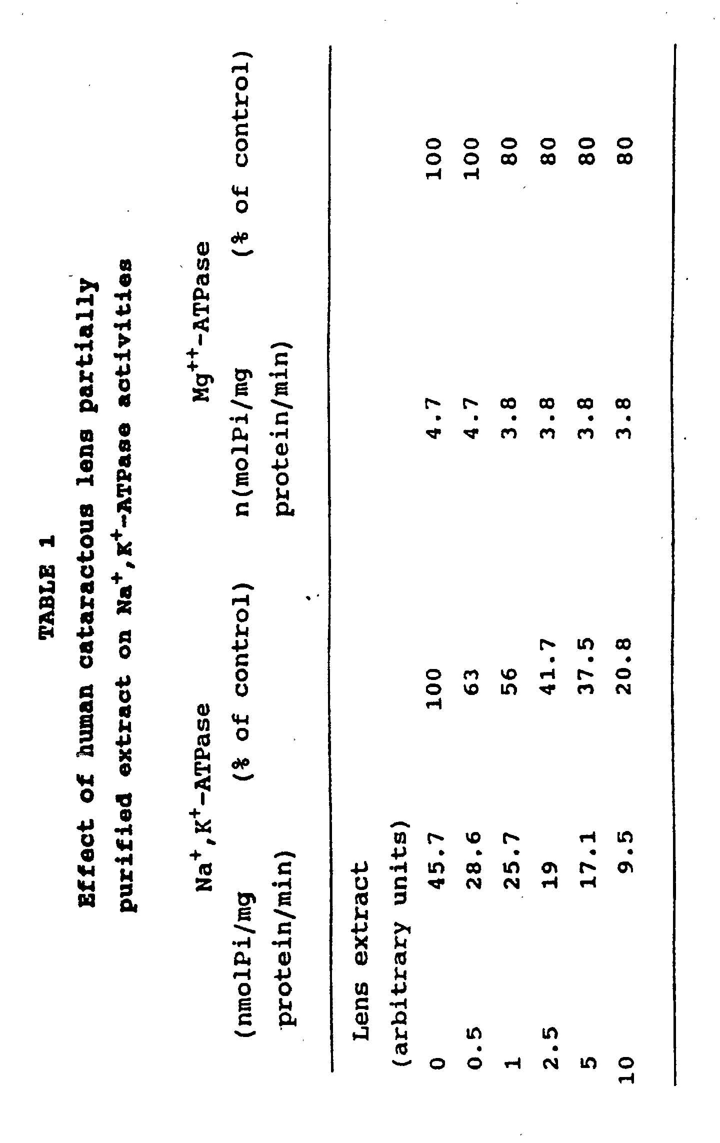

**TABLE 2**

| **Digoxin-like immunoreactivity in partially purified human cataractous lens extract** | | |
|---|---|---|
| Compound 10 µg/ml | Digoxin-like immunoreactive material,ng/ml | Cross reactivity % |
| Digoxin | 10,000 | 100 |
| Ouabain | 1.21 | 0.012 |
| Bufalin | 4.53 | 0.045 |
| Lens Extract | 2.26 | 0.022 |

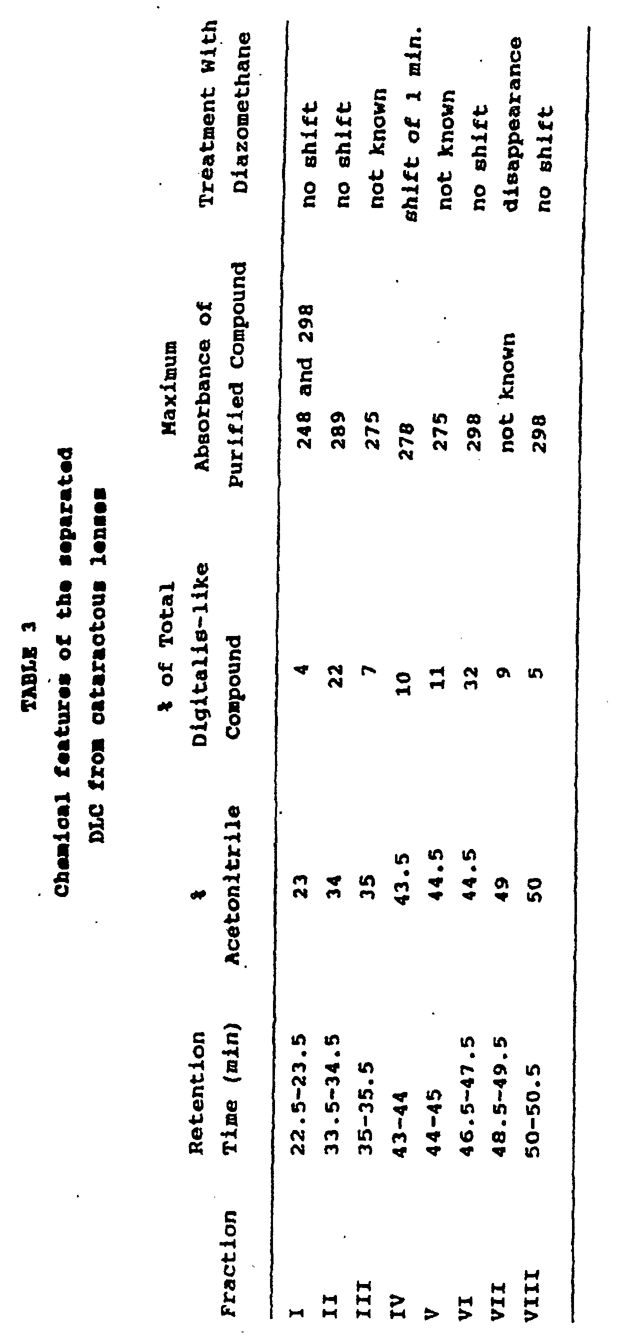

## Claims

1. A human cataractous lens nuclei methanolic extract consisting essentially of substances having a molecular weight lower than 3000 daltons, which substances are **characterised by** the following properties:
a) they are capable of inhibiting ATP hydrolysis by Na⁺, K⁺-ATPase;
b) they are capable of inhibiting ouabain binding to the ouabain binding site of Na⁺, K⁺-ATPase;
c) they are capable of cross-reacting with an antibody directed against digoxin; and
d) they have an absorption in the UV range.

2. A human cataractous lens nuclei extract according to claim 1, wherein said Na⁺, K⁺-ATPase is rat brain microsomal ATPase.

3. A compound of the general formula (I): 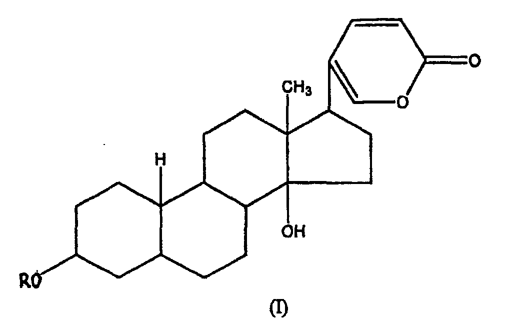 in which R is a hydrogen atom, an amino acid, a di- or tri-peptide or a mono-, di- or tri-saccharide.

4. A compound according to claim 3, wherein R is a hydrogen atom.

5. A compound according to claim 3, wherein:
a) said amino acid is threonine, glycine or alanine; or
b) said di- or tri-peptide consists of threonine, glycine or alanine residues; or
c) R is mannose.

6. A compound according to claim 3, wherein R is Thr-Gly-Ala-NH₂.

7. 3-hydroxy-14-en-bufa-20, 22- dienolide, or a conjugate thereof with a tri-peptide Thr-Gly-Ala-NH₂.

8. A method of preparing human cataractous lens nuclei extract according to claim 1 comprising the steps of:
a) homogenising a pool of human cataractous lens nuclei in methanol;
b) centrifuging the lens nuclei homogenate and removing the precipitate;
c) evaporating the methanolic fraction obtained in step (b) and dissolving the same in distilled water;
d) centrifuging the solution obtained in step (c) and collecting the aqueous supernatant;
e) subjecting said aqueous supernatant to chromatography on a solid phase extraction column, washing the column with water and eluting active material with 80% acetonitrile;
f) evaporating the acetonitrile and dissolving the residue in distilled water;
g) subjecting the solution obtained in step (f) to microconcentration and collecting the material passing <3000 daltons.

9. A pharmaceutical preparation comprising as an active ingredient a therapeutically effective amount of an extract according to claim 1 or 2, optionally further comprising a pharmaceutically acceptable carrier or diluent.

10. A pharmaceutical preparation comprising as an active ingredient a pharmaceutically effective amount of at least one compound according to any of claims 3 to 7, optionally further comprising a pharmaceutically acceptable additive, in a pharmaceutically acceptable carrier or diluent.

11. A pharmaceutical preparation according to claim 10, wherein said active ingredient is 19 norbufalin or its conjugate with Thr-Gly-Ala-NH₂.

12. Use of an extract according to claim 1 or 2, or of a compound according to any of claims 3 to 7, in the preparation of a medicament for the treatment or prevention of cardiac malfunction or renal malfunction.

13. Use of an extract according claim 1 or 2, or of a compound according to any of claims 3 to 7, in the preparation of a medicament for the induction of natriuresis and diuresis.

14. Use of an extract according claim 1 or 2, or of a compound according to any of claims 3 to 7, in the preparation of a medicament for modulating activity of the central nervous system.

## Patentansprüche

1. Methanolischer Extrakt aus humanen kataraktösen Linsenkernen, der im Wesentlichen aus Substanzen mit einem Molekulargewicht von weniger als 3000 Dalton besteht, wobei die Substanzen durch die folgenden Eigenschaften gekennzeichnet sind:
a) sie können die ATP-Hydrolyse durch Na⁺,K⁺-ATPase hemmen;
b) sie können die Bindung von Ouabain an die Ouabain-Bindungsstelle von Na⁺,K⁺-ATPase hemmen;
c) sie können eine Kreuzreaktion mit einem gegen Digoxin gerichteten Antikörper zeigen; und
d) sie weisen eine Absorption im UV-Bereich auf.

2. Extrakt aus humanen kataraktösen Linsenkernen gemäß Anspruch 1, wobei es sich bei der Na⁺,K⁺-ATPase um Rattenhirnmikrosomen-ATPase handelt.

3. Verbindung der allgemeinen Formel (I): 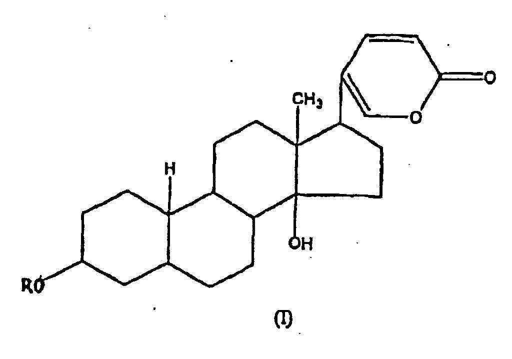 wobei R ein Wasserstoffatom, eine Aminosäure, ein Di- oder Tripeptid oder ein Mono-, Di- oder Trisaccharid ist.

4. Verbindung gemäß Anspruch 3, wobei R ein Wasserstoffatom ist.

5. Verbindung gemäß Anspruch 3, wobei:
a) es sich bei der Aminosäure um Threonin, Glycin oder Alanin handelt; oder
b) das Di- oder Tripeptid aus Threonin-, Glycin- oder Alaninresten besteht; oder
c) R Mannose ist.

6. Verbindung gemäß Anspruch 3, wobei R Thr-Gly-Ala-NH₂ ist.

7. 3-Hydroxy-14-en-bufa-20,22,dienolid oder ein Konjugat davon mit dem Tripeptid Thr-Gly-Ala-NH₂.

8. Verfahren zur Herstellung eines Extrakts aus humanen kataraktösen Linsenkernen gemäß Anspruch 1, umfassend die Schritte:
a) Homogenisieren eines Pools von humanen kataraktösen Linsenkernen in Methanol;
b) Zentrifugieren des Linsenkernhomogenisats und Entfernen des Sediments;
c) Eindampfen der in Schritt (b) erhaltenen methanolischen Fraktion und Auflösen derselben in destilliertem Wasser;
d) Zentrifugieren der in Schritt (c) erhaltenen Lösung und Gewinnen des wässrigen Überstands;
e) Chromatographieren des wässrigen Überstands auf einer Festphasen-Extraktionssäule, Waschen der Säule mit Wasser und Eluieren von aktivem Material mit 80%igem Acetonitril;
f) Abdampfen des Acetonitrils und Auflösen des Rückstands in destilliertem Wasser;
g) Mikrokonzentrieren der in Schritt (f) erhaltenen Lösung und Gewinnen des Materials, das < 3000 Dalton passiert.

9. Pharmazeutisches Präparat, das als Wirkstoff eine therapeutisch wirksame Menge eines Extrakts gemäß Anspruch 1 oder 2 umfasst und gegebenenfalls weiterhin einen pharmazeutisch annehmbaren Träger oder ein pharmazeutisch annehmbares Verdünnungsmittel umfasst.

10. Pharmazeutisches Präparat, das als Wirkstoff eine pharmazeutisch wirksame Menge wenigstens einer Verbindung gemäß einem der Ansprüche 3 bis 7 umfasst und gegebenenfalls weiterhin ein pharmazeutisch annehmbares Additiv in einem pharmazeutisch annehmbaren Träger oder Verdünnungsmittel umfasst.

11. Pharmazeutisches Präparat gemäß Anspruch 10, wobei es sich bei dem Wirkstoff um 19-Norbufalin oder sein Konjugat mit Thr-Gly-Ala-NH₂ handelt.

12. Verwendung eines Extrakts gemäß Anspruch 1 oder 2 oder einer Verbindung gemäß einem der Ansprüche 3 bis 7 bei der Herstellung eines Medikaments zur Behandlung oder Prävention einer Herzfehlfunktion oder Nierenfehlfunktion.

13. Verwendung eines Extrakts gemäß Anspruch 1 oder 2 oder einer Verbindung gemäß einem der Ansprüche 3 bis 7 bei der Herstellung eines Medikaments zur Induktion von Natriurese und Diurese.

14. Verwendung eines Extrakts gemäß Anspruch 1 oder 2 oder einer Verbindung gemäß einem der Ansprüche 3 bis 7 bei der Herstellung eines Medikaments zur Modulation der Aktivität des Zentralnervensystems.

## Revendications

1. Extrait méthanolique de noyaux de cristallin cataracté humain essentiellement constitué de substances ayant une masse moléculaire inférieure à 3 000 daltons, ces substances étant **caractérisées par** les 5propriétés suivantes :
a) elles sont capables d'inhiber l'hydrolyse de l'ATP par la Na⁺,K⁺-ATPase;
b) elles sont capables d'inhiber la liaison au strophantoside G au site de liaison au strophantoside G de la Na⁺,K⁺-ATPase ;
c) elles sont capables de réaction croisée avec un anticorps dirigé contre la digoxine ; et
d) elles ont une absorption dans le domaine UV.

2. Extrait de noyaux de cristallin cataracté humain selon la revendication 1, dans lequel ladite Na⁺,K⁺-ATPase est de l'ATPase microsomial de cerveau de rat.

3. Composé de formule générale (I) : 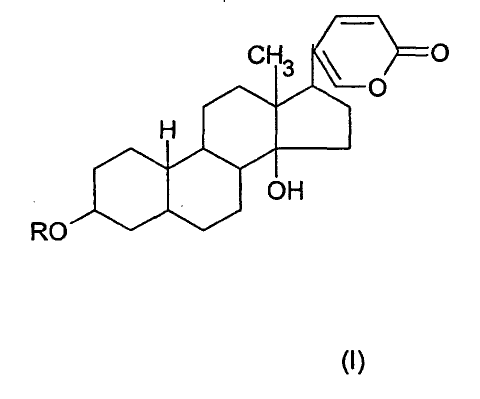 dans laquelle R est un atome d'hydrogène, un acide aminé, un di- ou tri-peptide, ou un mono-, di- ou tri-saccharide.

4. Composé selon la revendication 3, dans laquelle R est un atome d'hydrogène.

5. Composé selon la revendication 3, dans lequel :
a) ledit acide aminé est la thréonine, la glycine ou l'alanine ; ou
b) ledit di- ou tri-peptide se compose de résidus thréonine, glycine ou alanine ; ou
c) R est un mannose.

6. Composé selon la revendication 3, dans lequel R est Thr-Gly-Ala-NH₂.

7. 3-Hydroxy-14-ène-bufa-20,22-diénolide, ou un conjugué de ce composé avec un tripeptide Thr-Gly-Ala-NH₂.

8. Procédé de préparation d'extrait de noyaux de cristallin cataracté humain selon la revendication 1 comprenant les étapes consistant à :
a) homogénéiser un pool de noyaux de cristallin cataracté humain dans du méthanol ;
b) centrifuger l'homogénat de noyaux de cristallin et éliminer le précipité ;
c) évaporer la fraction méthanolique obtenue dans l'étape (b) et la dissoudre dans de l'eau distillée ;
d) centrifuger la solution obtenue dans l'étape (c) et recueillir le surnageant aqueux ;
e) soumettre ledit surnageant aqueux à une chromatographie sur une colonne d'extraction à phase solide, laver la colonne avec de l'eau et éluer la substance active avec de l'acétonitrile à 80% ;
f) évaporer l'acétonitrile et dissoudre le résidu dans de l'eau distillée ;
g) soumettre la solution obtenue dans l'étape (f) à une microconcentration et recueillir la matière traversant à <3 000 daltons.

9. Préparation pharmaceutique comprenant, comme ingrédient actif, une quantité efficace sur le plan thérapeutique d'un extrait selon la revendication 1 ou 2, éventuellement comprenant en outre un véhicule ou un diluant pharmaceutiquement acceptable.

10. Préparation pharmaceutique comprenant, comme ingrédient actif, une quantité efficace sur le plan pharmaceutique d'au moins un composé selon l'une quelconque des revendications 3 à 7, éventuellement comprenant en outre un additif pharmaceutiquement acceptable, dans un véhicule ou un diluant pharmaceutiquement acceptable.

11. Préparation pharmaceutique selon la revendication 10, dans laquelle ledit ingrédient actif est la 19-norbufaline ou son conjugué avec Thr-Gly-Ala-NH₂.

12. Utilisation d'un extrait selon la revendication 1 ou 2, ou d'un composé selon l'une quelconque des revendications 3 à 7, pour la préparation d'un médicament destiné au traitement ou à la prévention d'un dysfonctionnement cardiaque ou d'un dysfonctionnement rénal.

13. Utilisation d'un extrait selon la revendication 1 ou 2, ou d'un composé selon l'une quelconque des revendications 3 à 7, pour la préparation d'un médicament destiné à déclencher la natriurie et la diurèse.

14. Utilisation d'un extrait selon la revendication 1 ou 2, ou d'un composé selon l'une quelconque des revendications 3 à 7, pour la préparation d'un médicament destiné à moduler l'activité du système nerveux central.
